# EUROPEAN PATENT SPECIFICATION

(11) **EP 0 977 580 B1**
(45) Date of publication and mention of the grant of the patent: **09.04.2003**
(21) Application number: 98917411.5
(22) Date of filing: 20.04.1998
(51) Int. Cl.: A61K 38/17, C12N 5/10, G01N 33/50, C07K 16/18

(54) **MATERIALS AND METHODS RELATING TO INHIBITING THE INTERACTION OF p53 AND mdm2**
MATERIALEN UND VERFAHREN IM ZUSAMMENHANG MIT DER INHIBIERUNG DER WECHSELWIRKUNG DER P53 UND MDM2
MATERIELS ET PROCEDES RELATIFS A L'INHIBITION DE L'INTERACTION DE p53 ET mdm2

(30) Priority: 22.04.1997 GB 9708092
(43) Date of publication of application: 09.02.2000
(73) Proprietor: UNIVERSITY OF DUNDEE, Dundee DD1 4HN (GB)
(72) Inventor: LANE, David, Philip, St. Andrews,Fife KY16 O8N (GB)
(74) Representative: Kiddle, Simon John
(86) International application number: GB9801144
(87) International publication number: WO98047525

(56) References cited:
- WO-A-93/20238
- WO-A-96/02642
- WO-A-98/01467
- BLAYDES ET AL: "TOLERANCE OF HIGH LEVELS OF WILD-TYPE P53 IN TRANSFORMED EPITHELIAL CELLS DEPENDENT ON AUTO-REGULATION BY MDM-2" ONCOGENE, vol. 14, no. 15, 17 April 1997, pages 1859-1868, XP002075048 cited in the application
- BÖTTGER ET AL: "IDENTIFICATION OF NOVEL MDM2 BINDING PEPTIDES BY PHAGE DISPLAY" ONCOGENE, vol. 13, 1996, pages 2141-2147, XP002075049 cited in the application
- DATABASE MEDLINE FILE SERVER STN KARLSRUHE ABSTRACT 93390942, KOVAR ET AL: "NARROW SPECTRUM OF INFREQUENT P53 MUTATIONS AND ABSENCE OF MDM2 AMPLIFICATION IN EWING TUMOURS" XP002075050 & ONCOGENE, vol. 8, no. 10, October 1993, pages 2683-2690,

## Description

### Field of the Invention

The present invention relates materials and methods for inhibiting the interaction of p53 and mdm2, and more particularly to disrupting the binding of p53 and mdm2 or inhibiting the production of mdm2 in populations of cells that do not overexpress mdm2 to inhibit mdm2 targeting p53 for degradation.

### Background of the Invention

The tumour suppressor protein p53 is activated upon genotoxic insult to cells and acts as a transcription factor to induce cell cycle arrest or apoptosis in cells after DNA damage. Previous reports have suggested that cell proliferation might depend on a fine balance between expression of the oncogene mdm2 and the tumour suppressor p53 (Chen et al, 1994; Finlay, 1993; Otto and Deppert, 1993). This is due to an autoregulatory feedback loop for p53 activity involving mdm2, as mdm2 is transcriptionally activated by binding of p53 to an internal promoter within the mdm2 gene (Juven et al, 1993; Wu et al, 1993). It then binds the N-terminus of p53 thereby preventing p53 from interacting with the transcriptional machinery (Momand et al, 1992; Oliner et al, 1993).

The most impressive support for the importance of this autoregulatory loop in maintaining cell survival stems from experiments with transgenic mice. *Mdm2* -/- mice are only viable in the absence of p53 (Jones et al, 1995; Montes de Oca Luna et al, 1995). This argues that the level of p53 activity has to be tightly controlled in early development and that mdm2 is involved in this control pathway.

The cDNA sequence of human mdm2 is set out in WO 93/20238, which discloses that excess levels of mdm2 are present in some tumour cells such as certain types of sarcoma. This application discloses that the overexpression of mdm2 interferes with the normal feedback loop between mdm2 and p53, allowing cells overexpressing mdm2 to escape from p53-regulated growth control by binding p53. WO 93/20238 therefore suggests that molecules that inhibit the binding of p53 to mdm2 could be used as therapeutics for conditions in which mdm2 is overexpressed by alleviating this sequestration of p53, and thereby re-establishing normal p53 control. WO 93/20238 maps the domains of p53 that are necessary for mdm2 binding to amino acid residues 13-41, as well as additional residues on either the carboxy or the amino terminal side of this peptide.

WO 96/02642 describes experiments to refine the peptide motif of p53 responsible for binding to mdm2, and shows that the motif is less extensive than disclosed in WO 93/20238. WO 96/02642 discloses that a FxxLW motif between amino acid residues 18-23 of p53 (where x is any amino acid) is sufficient to bind to mdm2. This motif can be used to screen for therapeutic compounds capable of disrupting the interaction so that the transcriptional activity of p53 in cells overexpressing mdm2 can be restored.

Using phage display, a set of peptides that act as highly potent inhibitors in p53-mdm2 binding assays have been found based that contain mutations in the above mdm2 binding motif (Böttger et al, 1996).

However, a significant drawback in pursuing therapies based on this model of the role of mdm2 is that overexpression of mdm2 only occurs a small group of sarcomas, limiting the therapeutic applications of compounds found to be capable of disrupting the binding of p53 and mdm2.

### Summary of the Invention

The present invention is based on the finding that mdm2 binds to p53 in cells in which mdm2 is not overexpressed, i.e. in cells in which mdm2 is expressed at normal or low levels, and that in these cells, this interaction targets the p53 for degradation. This finding means that inhibiting mdm2 production and/or inhibiting the binding of mdm2 to p53 allows levels of p53 to increase by reducing the clearance of p53 by mdm2, and can be used to activate p53 function.

These results arose from experiments using highly potent peptide inhibitors of the interaction between mdm2 and p53 which were expressed as peptide aptamers on the surface of bacterial thioredoxin. Conventional peptides can have a very low potential to function *in vivo* because of their poor uptake and susceptibility to degradation. Accordingly, the experiments described herein are based on the construction of peptide aptamers presenting the peptide sequence of inhibitory peptides on the active site loop of *Escherichia coli* thioredoxin (LaVallie et al, 1993). These aptamers are characterised in mdm2 binding assays *in vitro.*

Microinjecting plasmids coding for these aptamers into cells containing p53 responsive reporter elements leads to striking activation of the β-galactosidase reporter gene. Cells with normal low levels of mdm2 respond even more dramatically than tumour cells which have accumulated high levels of mdm2.

The most potent aptamer, TIP 12/1, showed a similar binding affinity for mdm2 as bacterial full length wt p53. This made it a powerful inhibitor which could be expressed in mammalian cells.

Accordingly, in a first aspect, the present invention provides the use of an agent having the property of disrupting the binding of p53 and mdm2 or inhibiting the production of mdm2 in a population of cells, in the preparation of a medicament for activating p53, wherein the population of cells do not overexpress mdm2. Thus, the present invention is concerned with inhibiting the biological pathway by which mdm2 targets p53 for degradation in normal cells.

In a further aspect, the present invention provides a method of activating p53 comprising exposing a population of cells to an agent having the property of disrupting the binding of p53 and mdm2 or inhibiting the production of mdm2 so that p53 in the cells is activated, wherein the cells do not overexpress mdm2.

The p53 molecules that can be activated by the above method include wt p53 and mutant p53 molecules that retain the property of binding to mdm2, e.g through one or more of the interaction domains described in WO 93/20238 or WO 96/02642.

A variety of agents can be used to disrupt the binding of p53 and mdm2 or inhibit the production of mdm2 in a population of cells. Examples of the former type of agent include compounds comprising p53 peptide fragments having a mdm2 binding domain, or other compounds having the property of binding to one or more regions of mdm2 involved in binding p53, such as antibodies capable of blocking a p53 binding site of mdm2. Alternatively, compounds which compete with mdm2 for binding p53, but which do not inhibit a biological activity of p53, e.g. for DNA specific binding, can be used to inhibit mdm2 binding p53. Examples of these compounds include antibodies capable of blocking a mdm2 binding site of p53.

Examples of the latter approaches to limit mdm2 production include the use of antisense techniques to inhibit the production of mdm2, or the use of other substances that down regulate mdm2 production. These are discussed in detail below.

In a further aspect, the present invention provides a cell line that does not overexpress mdm2, the cell line being transfected with a reporter construct comprising nucleic acid encoding a reporter polypeptide under the control of promoter elements capable of responding to p53 activated for DNA specific binding to direct expression of the reporter polypeptide. Thus, this cell line can be used to test substances for the property of disrupting the binding of p53 and mdm2 or inhibiting the production of mdm2.

In a further aspect, the present invention provides a method of screening for substances capable of disrupting the binding of p53 and mdm2, or inhibiting the production of mdm2 in a population of cells that do not overexpress mdm2, the cells being transfected with a reporter construct comprising nucleic acid encoding a reporter polypeptide under the control of promoter elements that respond to the level of p53 activated for DNA specific binding to direct expression of the reporter polypeptide, the method comprising exposing the cells to the candidate substances and detecting the presence of the reporter polypeptide.

Conveniently, where the substance is a peptide, this can be achieved by transfecting the cells with an expression vector comprising nucleic acid encoding the substance so that the substance is expressed in cell transfected with the vector. As described below, the candidate peptide can be expressed as a fusion with a polypeptide such a thioredoxin, e.g. to display the candidate peptide in a particular conformation.

### Brief Description of the Figures

The present invention will now be described by way of example with reference to the accompanying figures. Further aspects of the present invention will be apparent to those skilled in the art.

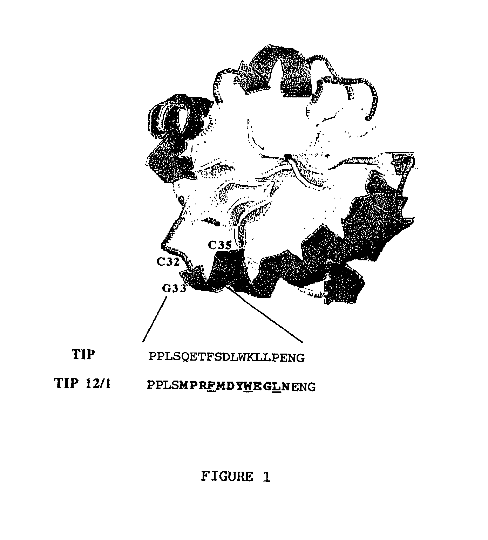

**Figure 1:** Schematic representation of the aptamers TIP and TIP 12/1 showing the peptide sequences inserted between G³³ and P³⁴ of E. coli thioredoxin. Deviations from the p53 wt sequence in TIP 12/1 are in bold with the non exchangeable amino acids underlined. The 3D structure for thioredoxin was obtained from the Protein Data Bank (PDB), Brookhaven National Laboratory and displayed using the public domain program RasMol.

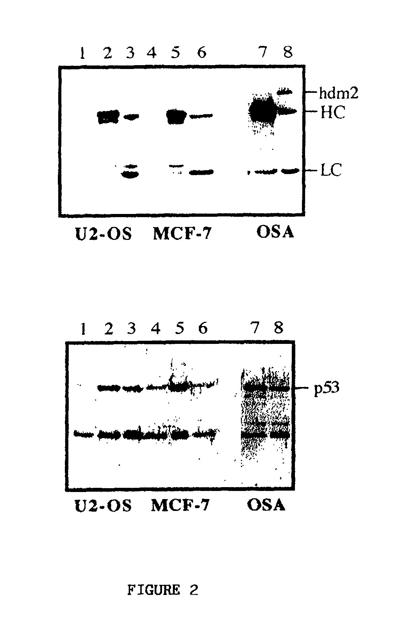

**Figure 2**: Immunoprecipitation from cellular lysates of U2-OS, MCF-7 and OSA cells using anti-p53 Pab 421 (lanes 2, 5 and 7), anti-mdm2 Mab 4B2 (lanes 3, 6 and 8) or no antibody for controls (lanes 1 and 4). Precipitated proteins were separated by SDS PAGE and Western blotted. In (a) Western blots were stained with a mixture of anti-mdm2 monoclonal antibodies (3G5, 4B2 and SMP 14). In (b) they were stained with anti p53 rabbit antiserum CM1(1/1000). The position of mdm2 in comparison with the heavy (HC) and light chain (LC) of mouse immunoglobulins are marked on the left hand side of the blot, also the position of p53. In lanes 1-8 on both blots, aliquots of the same samples were analysed.

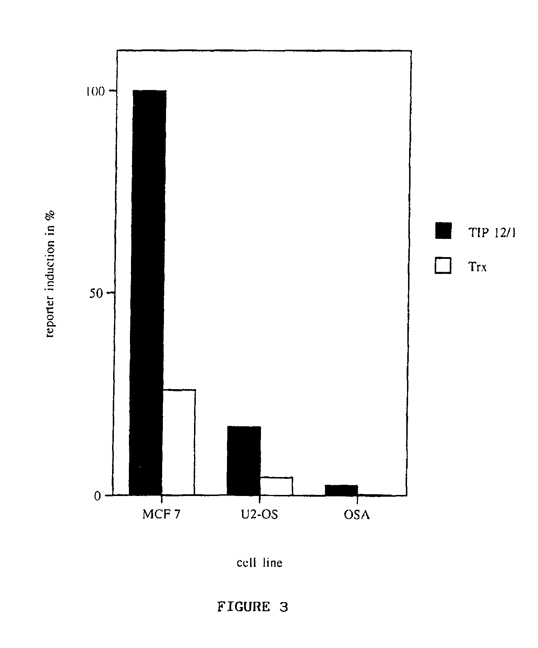

**Figure 3**: Soluble β-galactosidase assays of cell lysates transfected with RGCΔlacz and TIP 12/1 (black bars) or Trx (white bars) encoding DNA. The highest activity was measured in MCF-7 cells transfected with TIP 12/1 and set 100%.

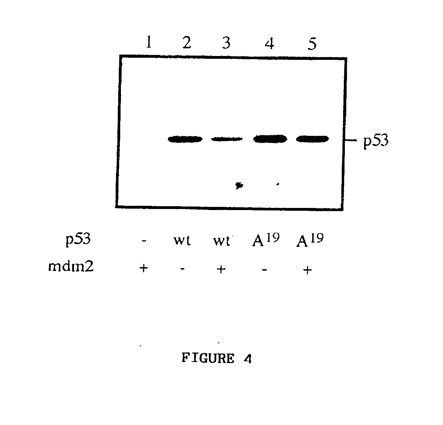

**Figure 4**: Western blot of SAOS 2 cell lysates 48 hrs after transfecting of control plasmid (lane 1), wt p 53 alone (land 2) or together with mdm2 (lane 3) and F¹⁹→A mutant p53 alone (lane 4) and in combination with mdm2 (lane 5).

### Detailed Description

In the present invention, "activating p53" refers to the property of activating p53 for DNA specific binding and transcription.

In the present invention, "cells that do not overexpress mdm2" includes all cells in which mdm2 is present at low or normal levels. This does not include cells in which mdm2 is overexpressed such as the sarcoma cells disclosed in WO 93/20238. It is possible to determine whether cells overexpress mdm2 by immunological measurement of mdm2 concentration, using methods familiar in the art.

### Peptides

One class of agents that can be used to disrupt the binding of p53 and mdm2 are peptides based on the sequence motifs of p53 that interact with mdm2. These peptides can be based on the regions of p53 that interact with mdm2 that are disclosed in WO 93/20238 and WO 96/02642. Such peptides tend to be small molecules, preferably less than 25 amino acids, more preferably less than 20 amino acids, more preferably less than 15 amino acids, and more preferably less than 10 amino acids in length. The present invention also encompasses peptides which are sequence variants or derivatives of a wild type p53 sequence.

Variant peptides have an amino acid sequence which differs from wt p53 sequence, e.g. in the motif between amino acids 13-41 described in WO96/02642, by one or more of addition, substitution, deletion and insertion of one or more amino acids, but which retains the activity of binding to mdm2. Such variants preferably include the motif FxxxW, where x is any amino acid, and will typically share at least about 70%, more preferably at least about 80%, more preferably at least about 90%, or more preferably at least about 95% amino acid sequence identity with the corresponding portion of human p53. Examples of peptides capable of disrupting the interaction of p53 and mdm2 and are the thioredoxin insert peptides (TIPs) disclosed in Böttger et al, 1996, and in the examples below, see especially peptide TIP 12/1.

The skilled person can use the techniques described herein and others well known in the art to produce large amounts of the peptides, or variants thereof, for use as pharmaceuticals or in the development of drugs. The peptides can be produced by expression from encoding nucleic acid and purified by techniques well known in the art.

The peptides can also be generated wholly or partly by chemical synthesis. The compounds of the present invention can be readily prepared according to well-established, standard liquid or, preferably, solid-phase peptide synthesis methods, general descriptions of which are broadly available (see, for example, in J.M. Stewart and J.D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984); and Applied Biosystems 430A Users Manual, ABI Inc., Foster City, California), or they may be prepared in solution, by the liquid phase method or by any combination of solid-phase, liquid phase and solution chemistry, e.g. by first completing the respective peptide portion and then, if desired and appropriate, after removal of any protecting groups being present, by introduction of the residue X by reaction of the respective carbonic or sulfonic acid or a reactive derivative thereof.

The invention also includes derivatives of the above peptides, comprising the peptide linked to a coupling partner, e.g. an effector molecule, a label, a drug, a toxin and/or a transport molecule such as the Penetratin peptide described in WO 91/19981. The techniques for coupling the peptides of the invention to both peptidyl and non-peptidyl coupling partners are well known in the art.

In addition, it is possible to express the peptide, peptide variant or derivative (where it is peptidyl) using an expression vector comprising nucleic acid encoding the peptide, variant or derivative under the control of control sequences to detect its expression.

Thus, where the substance or a part of it is peptidyl, a convenient way of producing it is to express nucleic acid encoding it in a suitable expression system. The use of expression system has reached an advanced degree of sophistication today.

Thus, the present invention also encompasses a method of making the substances disclosed herein, the method including expression from nucleic acid encoding the substance. This can conveniently be achieved by growing a host cell in culture, containing a vector comprising the nucleic acid under the control of sequences to direct its expression, under appropriate conditions which cause or allow expression of the peptide. Peptides may also be expressed in *in vitro* systems, such as reticulocyte lysate.

Systems for cloning and expression of a peptide in a variety of different host cells are well known. Suitable host cells include bacteria, eukaryotic cells such as mammalian and yeast, and baculovirus systems. Mammalian cell lines available in the art for expression of a heterologous peptide include Chinese hamster ovary cells, HeLa cells, baby hamster kidney cells, COS cells and many others. A common, preferred bacterial host is *E. coli.*

Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons, 1992.

A still further aspect provides a method which includes introducing the nucleic acid into a host cell. The introduction, which may (particularly for *in vitro* introduction) be generally referred to without limitation as "transformation", may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage. As an alternative, direct injection of the nucleic acid could be employed.

Marker genes such as antibiotic resistance or sensitivity genes may be used in identifying clones containing nucleic acid of interest, as is well known in the art.

The introduction may be followed by causing or allowing expression from the nucleic acid, e.g. by culturing host cells (which may include cells actually transformed although more likely the cells will be descendants of the transformed cells) under conditions for expression of the gene, so that the encoded peptide is produced. If the peptide is expressed coupled to an appropriate signal leader peptide it may be secreted from the cell into the culture medium. Following production by expression, a polypeptide may be isolated and/or purified from the host cell and/or culture medium, as the case may be, and subsequently used as desired, e.g. in the formulation of a composition which may include one or more additional components, such as a pharmaceutical composition which includes one or more pharmaceutically acceptable excipients, vehicles or carriers (e.g. see below).

### Antibodies

The agent used to disrupt the binding of p53 and mdm2 can be an antibody capable of specifically blocking a p53 binding site of mdm2, i.e. antibodies which are mdm2 antagonists. The production of antibodies is well known in the art and is described in detail below. An example of such an antibody is the 3G5 antibody described in Blaydes et al (1997).

The present invention also includes antibodies which compete with mdm2 for binding p53, but which do not inhibit a biological activity of p53, e.g. do not antagonise the DNA specific binding of p53.

It is possible to produce monoclonal antibodies to having the above binding specificities using techniques for doing this are well established in the art. Monoclonal antibodies can be subjected to the techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB-A-2188638 or EP-A-239400. A hybridoma producing a monoclonal antibody may be subject to genetic mutation or other changes, which may or may not alter the binding specificity of antibodies produced.

Preferred antibodies according to the invention are isolated, in the sense of being free from contaminants such as antibodies able to bind other polypeptides and/or free of serum components. Monoclonal antibodies are preferred for some purposes, though polyclonal antibodies are within the scope of the present invention.

Antibodies may be obtained using techniques which are standard in the art. Methods of producing antibodies include immunising a mammal (e.g. mouse, rat, rabbit, horse, goat, sheep or monkey) with the protein or a fragment thereof. Antibodies may be obtained from immunised animals using any of a variety of techniques known in the art, and screened, preferably using binding of antibody to antigen of interest. For instance, Western blotting techniques or immunoprecipitation may be used (Armitage et al, Nature, 357:80-82, 1992). Isolation of antibodies and/or antibody-producing cells from an animal may be accompanied by a step of sacrificing the animal.

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047. The library may be naive, that is constructed from sequences obtained from an organism which has not been immunised with any of the proteins (or fragments), or may be one constructed using sequences obtained from an organism which has been exposed to the antigen of interest.

Antibodies according to the present invention may be modified in a number of ways. Indeed the term "antibody" should be construed as covering any binding substance having a binding domain with the required specificity. Thus the invention covers antibody fragments, derivatives, functional equivalents and homologues of antibodies, including synthetic molecules and molecules whose shape mimics that of an antibody enabling it to bind an antigen or epitope.

Example antibody fragments, capable of binding an antigen or other binding partner are the Fab fragment consisting of the VL, VH, Cl and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

Humanised antibodies in which CDRs from a non-human source are grafted onto human framework regions, typically with the alteration of some of the framework amino acid residues, to provide antibodies which are less immunogenic than the parent non-human antibodies, are also included within the present invention.

A hybridoma producing a monoclonal antibody according to the present invention may be subject to genetic mutation or other changes. It will further be understood by those skilled in the art that a monoclonal antibody can be subjected to the techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB-A-2188638 or EP-A-0239400. Cloning and expression of chimeric antibodies are described in EP-A-0120694 and EP-A-0125023.

Hybridomas capable of producing antibody with desired binding characteristics are within the scope of the present invention, as are host cells, eukaryotic or prokaryotic, containing nucleic acid encoding antibodies (including antibody fragments) and capable of their expression. The invention also provides methods of production of the antibodies including growing a cell capable of producing the antibody under conditions in which the antibody is produced, and preferably secreted.

The reactivities of antibodies on a sample may be determined by any appropriate means. Tagging with individual reporter molecules is one possibility. The reporter molecules may directly or indirectly generate detectable, and preferably measurable, signals. The linkage of reporter molecules may be directly or indirectly, covalently, e.g. via a peptide bond or non-covalently. Linkage via a peptide bond may be as a result of recombinant expression of a gene fusion encoding antibody and reporter molecule.

### Regulation of mdm2 production in cells

The present invention include methods of regulating mdm2 production in cells that do not overexpress mdm2. An example of this approach is use of antisense techniques to inhibit the production of mdm2.

The antisense oligonucleotides can be based on the human mdm2 sequence set out in WO 93/20238, and used to inhibit or block the synthesis of mdm2 in cells in which mdm2 is expressed at normal or low levels. Antisense oligonucleotides may be designed to hybridise to the complementary sequence of nucleic acid, pre-mRNA or mature mRNA, interfering with the production of polypeptide encoded by a given DNA sequence, so that its expression is reduce or prevented altogether. In addition to the mdm2 coding sequence, antisense techniques can be used to target the control sequences of the mdm2 gene, e.g. in the 5' flanking sequence of the mdm2 coding sequence, whereby the antisense oligonucleotides can interfere with mdm2 control sequences. The construction of antisense sequences and their use is described in Peyman and Ulman, Chemical Reviews, 90:543-584, (1990), Crooke, Ann. Rev. Pharmacol. Toxicol., 32:329-376, (1992), and Zamecnik and Stephenson, P.N.A.S, 75:280-284, (1974).

The cell line mentioned above can be used to screen for substances having the property of modulating mdm2 production, e.g. reducing the amount of mdm2 expressed in the cells to reduce the targeting of p53 for degradation. This is discussed further below.

### Pharmaceutical compositions

The agents of the invention can be formulated in pharmaceutical compositions, e.g. as medicaments for activating p53 in cells that do not overexpress mdm2. These compositions may comprise, in addition to one of the above substances, a pharmaceutically acceptable excipient, carrier, buffer, stabiliser or other materials well known to those skilled in the art. Such materials should be nontoxic and should not interfere with the efficacy of the active ingredient. The precise nature of the carrier or other material may depend on the route of administration, e.g. oral, intravenous, cutaneous or subcutaneous, nasal, intramuscular, intraperitoneal routes.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Whether it is a polypeptide, antibody, peptide, nucleic acid molecule, small molecule or other pharmaceutically useful compound according to the present invention that is to be given to an individual, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980.

Alternatively, targeting therapies may be used to deliver the active agent more specifically to certain types of cell, by the use of targeting systems such as antibody or cell specific ligands. Targeting may be desirable for a variety of reasons; for example if the agent is unacceptably toxic, or if it would otherwise require too high a dosage, or if it would not otherwise be able to enter the target cells.

Instead of administering these agents directly, they could be produced in the target cells by expression from an encoding gene introduced into the cells, eg in a viral vector (a variant of the VDEPT technique - see below). The vector could be targeted to the specific cells to be treated, or it could contain regulatory elements which are switched on more or less selectively by the target cells.

Alternatively, the agent could be administered in a precursor form, for conversion to the active form by an activating agent produced in, or targeted to, the cells to be treated. This type of approach is sometimes known as ADEPT or VDEPT; the former involving targeting the activating agent to the cells by conjugation to a cell-specific antibody, while the latter involves producing the activating agent, e.g. an enzyme, in a vector by expression from encoding DNA in a viral vector (see for example, EP-A-415731 and WO 90/07936).

A composition may be administered alone or in combination with other treatments, either simultaneously or sequentially dependent upon the condition to be treated, such as cancer, virus infection or any other condition in which p53 or mdm2 is not functioning.

### Methods of Screening for Drugs

The cell line described above, which does not overexpress mdm2 and which is transfected with a reporter construct comprising nucleic acid encoding a reporter molecule under the control of promoter elements that respond to the level of p53 activated for DNA specific binding, can be used to screen candidate substances for the biological property of disrupting the interaction of p53 and mdm2 or inhibiting mdm2 production. Thus, if a candidate substance has this property, it will lead to p53 to become available in the cells for DNA specific binding and cause expression of the nucleic acid encoding the reporter. Examples of suitable cell types include human fibroblasts, keratinocytes and tumour cell lines expressing wild-type p53. Examples of reporter include β-galactosidase, luciferase, chloramphenicol acetyl transferase and green fluorescent protein.

The identification of substances having one of the above mentioned biological properties can be used in the rational design of therapeutics as discussed below.

It is well known that pharmaceutical research leading to the identification of a new drug may involve the screening of very large numbers of candidate substances, both before and even after a lead compound has been found. This is one factor which makes pharmaceutical research very expensive and time-consuming. Means for assisting in the screening process, such as the above cell lines, can have considerable commercial importance and utility.

In the screening process, combinatorial library technology provides an efficient way of testing a potentially vast number of different substances for property of activating p53 in cells that do not overexpress mdm2.

Candidate substances can also be screened for the ability to interact with the mdm2, e.g. in a yeast two-hybrid system (which requires that both the polypeptide and the test substance can be expressed in yeast from encoding nucleic acid). This may be used as a coarse screen prior to testing a substance for the property of causing p53 activation.

Following identification of a substance which modulates or affects polypeptide activity, the substance may be investigated further. Furthermore, it may be manufactured and/or used in preparation, i.e. manufacture or formulation, of a composition such as a medicament, pharmaceutical composition or drug. These may be administered to individuals.

Thus, the present invention extends in various aspects not only to a substance identified using a nucleic acid molecule as a modulator of polypeptide activity, in accordance with what is disclosed herein, but also a pharmaceutical composition, medicament, drug or other composition comprising such a substance, a method comprising administration of such a composition to a patient, e.g. for treatment (which may include preventative treatment) of cancer and other hyperproliferative disorders, use of such a substance in manufacture of a composition for administration, and a method of making a pharmaceutical composition comprising admixing such a substance with a pharmaceutically acceptable excipient, vehicle or carrier, and optionally other ingredients.

A substance identified using as a modulator of polypeptide function may be peptide or non-peptide in nature. Non-peptide "small molecules" are often preferred for many *in vivo* pharmaceutical uses. Accordingly, a mimetic or mimic of the substance (particularly if a peptide) may be designed for pharmaceutical use.

The designing of mimetics to a known pharmaceutically active compound is a known approach to the development of pharmaceuticals based on a "lead" compound. This might be desirable where the active compound is difficult or expensive to synthesise or where it is unsuitable for a particular method of administration, e.g. peptides are unsuitable active agents for oral compositions as they tend to be quickly degraded by proteases in the alimentary canal. Mimetic design, synthesis and testing is generally used to avoid randomly screening large number of molecules for a target property.

There are several steps commonly taken in the design of a mimetic from a compound having a given target property. Firstly, the particular parts of the compound that are critical and/or important in determining the target property are determined. In the case of a peptide, this can be done by systematically varying the amino acid residues in the peptide, e.g. by substituting each residue in turn. Alanine scans of peptide are commonly used to refine such peptide motifs. These parts or residues constituting the active region of the compound are known as its "pharmacophore".

Once the pharmacophore has been found, its structure is modelled to according its physical properties, eg stereochemistry, bonding, size and/or charge, using data from a range of sources, e.g. spectroscopic techniques, X-ray diffraction data and NMR. Computational analysis, similarity mapping (which models the charge and/or volume of a pharmacophore, rather than the bonding between atoms) and other techniques can be used in this modelling process.

In a variant of this approach, the three-dimensional structure of the ligand and its binding partner are modelled. This can be especially useful where the ligand and/or binding partner change conformation on binding, allowing the model to take account of this in the design of the mimetic.

A template molecule is then selected onto which chemical groups which mimic the pharmacophore can be grafted. The template molecule and the chemical groups grafted on to it can conveniently be selected so that the mimetic is easy to synthesise, is likely to be pharmacologically acceptable, and does not degrade *in vivo*, while retaining the biological activity of the lead compound. Alternatively, where the mimetic is peptide based, further stability can be achieved by cyclising the peptide, increasing its rigidity. The mimetic or mimetics found by this approach can then be screened to see whether they have the target property, or to what extent they exhibit it. Further optimisation or modification can then be carried out to arrive at one or more final mimetics for *in vivo* or clinical testing.

### Experimental Procedures

### Cloning and expression of peptide aptamers

pTrx (Invitrogen) was cleaved with RsrII. The following oligomers were phosphorylated, annealed and then ligated into the cleaved vector:

For TIP wt: 5'-3'
GTCCGCCTCTGAGTCAGGAAACATTTTCAGACCTATGGAAACTACTTCCTGAAAACG

and 5'-3'
GACCGTTTTCAGGAAGTAGTTTCCATAGGTCTGAAAATGTTTCCTGACTCAGAGGCG

For TIP 12/1: 5'-3'
GTCCGCCTCTGAGTATGCCTCGTTTTATGGATTATTGGGAGGGTCTTAATGAAAACG

and 5'-3'
GACCGTTTTCATTAAGACCCTCCCAATAATCCATAAAACGAGGCATACTCTCAGAGGCG

The resulting peptide inserts are illustrated in Figure 1.

*E. coli* 1724 cells were transformed with the resulting plasmids. They were grown in RM medium at 30°C overnight, then inoculated into fresh induction medium and grown to OD 0.5. The cultures were transferred to 37°C, induced with L-tryptophan at a final concentration of 100 µg/ml and grown for three to four hours. Soluble extracts were obtained by resuspending pellets in ice cold 20 mM Tris/HCl, pH 8, 2.5 mM EDTA with protease inhibitors (1 mM PMSF, 1mM benzamidine, leupeptin, approtinin and pepstatin at 10 µg/ml each) and three times shock freezing, thawing and sonicating, followed by centrifugation for 20 min at 10,000 g. Heat shock lysates were obtained by resuspending pellets to an OD of 100 and then treating at 80°C for 10 min followed by centrifugation at 10,000g for 20 min.

Purification of soluble extracts was carried out by loading clear soluble lysates onto an ion exchange Q 50 column (BioRad) and eluting with a linear gradient of 0.05M-1M KCl in 50 mM Tris/HCl pH 7.8, 0.1% Triton X-100, 10% glycerol and 50 mM KCl. Active fractions were identified with anti Thioredoxin antibody (Invitrogen) on dot blots, concentrated using Centriprep 3 filters (Amicon) and loaded onto a G100 column (Pharmacia), preequilibrated with 30 mM HEPES, pH 8.0, 500 mM KCl, 0.1% Triton X100, 10% glycerol. Active fractions after elution were pooled, concentrated and dialysed against PBS.

For cloning of TIP 12/1, TIP wt and Trx into pcDNA3 for expression in mammalian cells, the thioredoxin coding region complete with the peptide insertions, was amplified from pTrx, pTrx 12/1 and pTrx wt using the following primers:

5′-3′: CGGGATCCACCATGGGCGATAAAATTATTCACCTG and 5′-3′
CTCGACGCTAACCTGGCCTAGGGAATTCC.

The resulting PCR products were cleaved with BamHI and Eco RI and ligated into BamHI, EcoRI cleaved pcDNA3. The TIP 12/1 sequence in pcDNA3 was verified by sequencing.

### ELISA assays

ELISAs were carried out as previously described (Böttger et al, 1996). Briefly, plates were coated with 1µg/ml p53 or dilutions of p53 overnight at 4°C. They were blocked and a preincubated mixture of GST-hdm2 (1.3 µg/ml) and synthetic peptides (inhibition ELISA) or hdm2 alone (direct binding ELISA) was added for 1 hr. Binding was established with the anti mdm2 monoclonal antibody SMP 14 (Picksley et al, 1994) and HRP conjugated anti-mouse IgG.

### Cell culture and microinjection

All cells were grown in Dulbecco's modified Eagle medium (DMEM) supplemented with 10% FCS, for T22 cells 1 mg/ml G418 was added.

For microinjection, cells were seeded onto tissue culture dishes and grown to 60-70% confluence. Microinjection was performed using an Eppendorf microinjection system (Microinjector 5242, Micromanipulator 5170) mounted to an Axiovert 35 M with heated stage. Antibody injections were intranuclear or cytoplasmic. Plasmid injections were intranuclear. Purified mouse monoclonal antibodies 3G5 and 4B2 were injected in PBS at a concentration of ca 1.3 mg/ml. Plasmid DNA encoding for TIP 12/1, TIP and Trx was purified using Quiagen purification system or phenol/chloroform precipitation and injected at a concentration of 0.25 mg/ml in water. Following microinjection fresh medium was added to the cell cultures and they were incubated for 24 hours.

### Detection of β-galactosidase activity

Cells were washed with PBS and fixed with 2% formaldehyde/0.2% glutaraldehyde in PBS for 5 min on ice. They were again washed with PBS and overlaid with 0.25 mg/ml X-gal in reaction mix (5 mM potassium ferricyanide, 5 mM potassium ferrocyanide, 2 mM magnesium chloride in PBS). Cells were incubated at 37°C for 16 hours. Blue stained cells were observed.

### Immunofluorescence

VRn.6 cells were fixed with 4% paraformaldehyde in PBS for 10 min, washed and permeabilised with 1% NP40 in PBS. Primary antibodies rabbit anti β-galactosidase or monoclonal anti-thioredoxin antibody (Anti-Thio™ Antibody, Invitrogen) were applied 1/500, Texas red conjugated goat anti mouse Ig or FITC conjugated goat and rabbit Ig F(ab')₂ fragments (Jackson) were applied 1/500 as second antibodies. Incubations were carried out for 1 hour or 45 min at RT. Washes were carried out in PBS.

T22 cells were fixed with ice cold acetone/methanol (½) for 8 min at 4°C. Primary antibodies, protein A purified CM5 at 2µg/ml or anti-Thio™ 1/500 were added for 1 hour at RT, after PBS washes FITC conjugated anti mouse Ig at 1/80 or Texas red anti rabbit Ig at 1/400 were added for 30 min at RT.

### Transient transfections for reporter induction

Cells were seeded into 6 well plates at 1.5 x 10⁶ cells per well. They were grown to a density of 80% confluence and transfected using different lipophilic reagents (lipofectin and lipofectamin, Promega, DOSPER and DOTAP, Boehringer). 2.5 µg TIP encoding plasmid DNA and 1 µg RGCΔFosLacZ DNA and 5 - 10 µg of lipophilic reagent according to the instructions of the manufacturers were mixed in serum free medium and applied to the cells. 2-4 hours after transfection complete medium was added. 48 hours after transfection β-galactosidase activity was measured using CPRG (Boehringer) as a substrate. Cells were scraped into PBS and centrifuged. Pellets from each well were dissolved in 50 µl Reporter Lysis buffer (Promega) and incubated on ice for 15 min. Soluble lysates were incubated with CPRG in 100 mM phosphate buffer, pH 7.0. OD at 595 nm was measured 1 - 24 hours later. To measure transfection efficiencies in each experiment, cells in a separate well were transfected with 2.5 µg pG3 DNA, encoding firefly luciferase. Luciferase activity was measured using Promegas luciferase assay system. The same lysates served as control for endogenous β-galactosidase activity.

### Immunoprecipitations

Cells were grown to > 90% confluence on 14 cm dishes. They were scraped into PBS and lysed in 50 mM Tris/HCl, pH 8.0, 150 mM NaCl, 5 mM EDTA, 0.5% NP 40 and protease inhibitors (Boehringer Complete™). To 100 µl lysate 1 µg PAb 421 in 400 µl DMEM/10% FCS or 400 µl Mab 4B2 supernatant was added and the lysates incubated overnight at 4°C. Protein G sepharose beads were added and incubation carried out for 2 hours. Beads were washed 5 times with PBS, 0.2% Tween and then boiled in SDS sample buffer. Proteins were separated on PAGE gels, western blotted and blots stained with rabbit polyclonal anti-p53 antibodies CM5 and CM1 followed by HRP anti rabbit IgG or a mixture of anti-mdm2 monoclonal antibodies 3G5, SMP14 and 4B2 followed by HRP anti-mouse IgG (DAKO). HRP activity was established by ECL (Amersham).

### Transient transfections for establishing p53 levels in the presence and absence of mdm2

SAOS 2 cells were seeded 24 hrs before transfection to 80% confluence in 10 cm dishes. Calcium phosphate mediated transfections were performed as previously described (Lin and Green, 1989). 5 µg of p53 wt and mutant encoding plasmids (pcDNA3) or control vector and 5 µg mdm2 encoding plasmid (X2, Haupt et al., 1996) were cotransfected per 10 cm dish. 48 hrs after transfection cell lysates were fractionated by 12% SDS-PAGE and transferred to nitrocellulose membranes. Blots were stained with rabbit anti p53 antiserum CM5 (1/8000) and HRP conjugated anti rabbit Ig. Peroxidase activity was established using ECL.

### Site directed mutagenesis

Construction of F¹⁹→A was accomplished by site directed mutagenesis using the Transformer™ site directed mutagenesis kit (Clonetech). The sequence of the selection primer was: 5'-3' GACTCTGGGGATCGATATGACCGACC, the sequence of the mutagenic primer was: 5'-3' GAGCCAGGAGACAGCCTCAGGCTTATG. The sequence of the p53 mutant F¹⁹→A was confirmed by sequencing.

### Results

### Peptide inserts into Thioredoxin create potent inhibitors of the p53-mdm2 interaction.

Figure 1 shows a schematic representation of the three aptamers we constructed by inserting additional peptide sequences into the active site of E.coli thioredoxin. TIP 12/1 (Thioredoxin Insert Protein) contains the sequence we identified previously by phage display as the most potent inhibitor of the mdm2-p53 interaction in in vitro assays (Böttger et al, 1996). TIP wt contains the sequence corresponding to p53 wild type sequence p13 to N²⁹. As controls we expressed thioredoxin lacking a peptide insertion (Trx) in bacteria.

All these proteins were easily expressed in *E. coli.* They could be purified from soluble bacterial lysates by heat shock to almost 80% homogeneity without loss of mdm2 binding activity. We also purified the TIPs to near homogeneity from soluble bacterial lysates which were not heat shocked by ion exchange chromatography and gel filtration. Results obtained with TIPs purified both ways were identical.

We tested the aptamers for their capacity to inhibit the p53-mdm2 interaction in ELISA assays. Binding of GST-mdm2 (1-188) to p53 coated ELISA plates was inhibited by increasing amounts of bacterially expressed TIPs. Table 1 shows IC₅₀ values obtained in this assay in comparison with the IC₅₀ for the corresponding peptides and bacterially expressed full length p53 (Midgley et al, 1992). The following conclusions can be drawn.
1. TIP 12/1 inhibits the interaction between p53 and mdm2 in this assay with the same strength as full length p53. This should make it a suitable agent to be tested in cellular systems for effects on the interaction between p53 and mdm2 *in vivo.*
2. TIP wt inhibits the interaction 20 times less than TIP 12/1. This has to be attributed to the 50 times less potent inhibition achieved by the wt peptide when compared with peptide 12/1 in peptide competition assays (Böttger et al, 1997).
3. Trx does not show inhibition in our assay, making it a suitable negative control for *in vivo* experiments.

In these *in vitro* assays, TIP 12/1 exhibits strong enough inhibitory potential to compete against endogenous levels of wt p53 in tumour cells for binding to mdm2. It therefore offers an agent that should be capable to function inside mammalian cells.

### Expression of peptide aptamers in mammalian cells activates p53 dependent transcription.

We therefore went on to clone TIP 12/1, TIP wt and Trx into pcDNA3 (Promega), a vector where these proteins would be expressed under the control of the strong CMV promoter in mammalian cells.

At first we wanted to see if a stably transfected p53 responsive β-galactosidase reporter could be switched on by microinjecting the plasmids encoding for TIPs. A recently established transformed rat thyroid epithelial cell line stably transfected with pRGCΔ fos-lac Z (Blaydes et al, 1997), VRn.6 cells, seemed to provide a suitable model. It was shown before that these cells express wt p53 and also overexpress mdm2 at the protein level. The β-galactosidase reporter is strongly responsive to UV induction of p53 in VRn.6 cells. Microinjection of the monoclonal antibody 3G5 into the nuclei of these cells could also switch on the p53 reporter (Blaydes et al, 1997). We have shown before that 3G5 binds mdm2 exactly within the p53 binding pocket and blocks p53 mdm2 association (Böttger et al, 1997). If the effect of 3G5 injection on the induction of p53 dependent transcription was due to interrupting the p53-mdm2 interaction, our inhibiting aptamers should exert a similar effect.

In these experiments we found the strong induction of β-galactosidase activity after microinjection of 3G5 into the nuclei of VRn.6 cells. To be certain that the cells which responded with β-galactosidase activity were the injected ones we carried out dual immunofluorescence studies. We stained cells after injections with anti β-galactosidase antiserum and with anti mouse immunoglobulin. This showed that the cells expressing the reporter enzyme (left hand panel, blue) are also positive for the injected antibody (right hand panel, green). Injecting TIP 12/1 encoding plasmid into the nuclei of VRn.6 cells also has a strong effect on induction of the reporter enzyme. There is no induction after microinjection of the control thioredoxin encoding plasmid, although it clearly is expressed.

The remarkable strength of this response lead us to test whether this was due to the relatively high levels of p53 and mdm2 present in the VRn.6 line. To do this the microinjection experiments were then carried out in T22 cells, a mouse prostate derived cell line, also stably transfected with the same reporter plasmid. These cells normally contain very low levels of p53 and mdm2. On treatment with DNA damaging agents the p53 protein accumulates and the cells show remarkable p53-dependent β-galactosidase induction (Hupp et al, 1995; Lu and Lane, 1993). On microinjection of 3G5 and TIPs encoding plasmids into T22 cells, we again detect immense induction of β-galactosidase with 3G5, but no induction with 4B2, an anti mdm2 antibody that targets an epitope outside the p53 binding pocket on mdm2. A remarkable reporter induction was caused by our strongest aptamer, TIP 12/1. Lower levels of β-galactosidase activity are observed with TIP and no activity with the control thioredoxin. This reflects exactly the capacity of the three aptamers to inhibit the mdm2-p53 interaction in vitro. Staining of the injected cells with anti-thioredoxin antibody confirmed that the differences in reporter enzyme activity were not due to differential expression of the TIP proteins.

The strong induction of reporter enzyme in T22 cells, which do not overexpress mdm2, lead us to believe, that activation of p53 by release from mdm2 complexes must be a very strong stimulus for p53 activation, similar to induction of p53 by UV and much stronger than the previously observed effect of allosteric activation of latent p53 in T22 cells by PAb 421 (Hupp et al, 1995).

We sought to analyse more cell lines, preferentially not dependent on a stably integrated reporter plasmid. We therefore transiently transfected cells containing wt p53 with p53 responsive reporter plasmids (RGCΔFosLacZ) and TIP 12/1 or Trx control. We choose OSA cells, a human osteosarcoma cell line (Florenes et al, 1994) as an example for a cell line with highly elevated mdm2 levels due to gene amplification. We also used U2-OS cells, another osteosarcoma cell line, which has no gene amplification for mdm2 but elevated levels of mdm2-mRNA (Florenes et al, 1994) and MCF-7 cells, a breast cancer cell line with heterogeneously expressed low levels of wt p53 and no reported mdm2 elevation. First we tried to analyse the protein levels expressed in these cells and the degree of complex formation with mdm2, using immunoprecipitations. We precipitated from cell lysates with the monoclonal anti-mdm2 antibody 4B2 and the monoclonal anti-p53 antibody 421. Figure 2 shows the results.

In OSA cells, 4B2 coprecipitates p53 in similar amounts to PAb 421 (Fig 2b, lane 7 for PAb 421 and lane 8 for 4B2). 4B2 precipitates mdm2 (Fig 2a, lane 7), suggesting an excess of mdm2 that does not bind p53 whilst all p53 is in complex with mdm2. In U2-OS and MCF-7 cells neither 4B2 nor PAb 421 precipitate readily detectable amounts of mdm2 (Fig 2a, lanes 2 and 5 for PAb 421, lanes 3 and 6 for 4B2, lanes 1 and 4 represent controls for precipitations without antibodies). After superexposure of ECL film for 60 min, a very faint band at the size of mdm2 appears in U2-OS cell precipitates (not shown). In MCF-7 cells PAb 421 precipitates p53 (Fig 2b, lane 5), but no coprecipitation of p53 by anti-mdm2 antibody 4B2 is observed (Fig 2b, lane 6, signal is the same as in control precipitation, lane 4). In U2-OS cells PAb 421 precipitates p53 (Figure 2b, lane 3) and 4B2 precipitates a large percentage of p53 (Figure 2b, lane 3), suggesting a high degree of hdm2-p53 complex formation.

We then cotransfected (RGCΔFosLacZ) reporter plasmid and TIP 12/1 or Trx encoding DNA into all three cell lines to compare the levels of reporter enzyme induction directly. Different lipophilic transfection agents were used and the transfection efficiency was monitored on separate plates by transfecting pG3, a plasmid encoding firefly luciferase under a constitutive promoter, and measuring luciferase activity. This allowed us to exclude the possibility that the differences in p53 dependent transcriptional activation of the reporter are related to transfection efficiency (data not shown). Figure 3 shows an average of four experiments of induction of β-galactosidase activity by TIP 12/1, compared to Trx in each cell line.

Surprisingly, most induction of the p53 reporter is achieved by TIP 12/1 in MCF-7 cells and in U2-OS cells, where the level of mdm2 is below the detection limit. A ca. 100 times lower effect of reporter enzyme induction TIP 12/1 is observed in OSA cells. Figure 3 also shows, that transfection of control plasmid alone induces a low level response of p53 dependent transcriptional activation in MCF-7 and U2-OS cells. This effect has been reported before (Renzing and Lane, 1993). It is, however, almost completely absent in OSA cells.

This confirms the concept, that in cells overexpressing mdm2 by gene amplification, p53 dependent transcriptional activation is repressed. This can be overcome by inhibiting the interaction between mdm2 and p53. Much more striking, however, is the observation that in cells with undetectable levels of mdm2, specific inhibitors of the p53-mdm2 interaction can induce p53 dependent transcriptional activation to an extent that is comparable with induction by UV.

### Disruption of the p53-mdm2 interaction leads to the accumulation of p53

The dramatic induction of the p53 response by UV and other genotoxic agents is accompanied by the accumulation of high levels of p53 protein due at least in part to its extended half life in treated cells. However, it is not clear that this is the sole mechanism by which the p53 response is activated an other mechanisms such as allosteric activation of DNA binding function have also been proposed to play a role (Hupp et al, 1995). The very strong induction of the p53 response by the TIP 12/1 encoding plasmid and MAb 3G5 injections lead us to ask if this induction occurred independently of the accumulation of p53 protein. To test this hypothesis, Mabs 3G5 and 4B2 and TIP 12/1 and Trx encoding plasmids were injected into T22 cells and the levels of p53 analysed in immunofluorescence. We found that p53 accumulates to high levels in the cells injected with the interaction disrupting 3G5 antibody, but not with the control anti-mdm2 antibody. In a similar way, cells injected with the TIP 12/1 expression plasmid accumulate high levels of p53, whereas those injected with control plasmid do not. These striking results demonstrate that disruption of the p53-mdm2 interaction mirrors the genotoxic response not only by activating p53 dependent transcription, but also by leading to the accumulation of p53 protein. The implication of these results was that in normal cells mdm2 targets p53 for destruction. When we transfected wild type p53 into p53 negative SAOS 2 cells we found that cotransfection of an mdm2 expression plasmid greatly reduced the level of p53 that accumulated (Figure 4, lanes 2 and 3), supporting the idea that mdm2 could target p53 for degradation. To further test this hypothesis we constructed a point mutant F¹⁹→A in murine p53 that mutated one of the key contact residues of the p53-mdm2 interface identified in the crystal structure and in our phage display analysis (Böttger et al, 1997; Kussie et al, 1996). We confirmed that this mutant p53 was unable to bind to mdm2 but was transcriptionally active (data not shown). When this mutant p53 was transfected into the SAOS 2 cells its accumulation unlike that of wild type p53 was not affected by cotransfection of the mdm2 expression plasmid (Figure 4, lanes 4 and 5).

### Discussion

### Peptide aptamers to disrupt protein protein interactions

The above results show that peptide aptamers able to block the binding of p53 to mdm2 in cellular assays. To do this peptide sequences capable of binding tightly to mdm2 that had been identified from phage peptide libraries were displayed on the active site loop of thioredoxin. The aptamer proteins (TIPs) were readily expressed in *E.coli* and easily purified from soluble lysates. In *in vitro* assays the peptides inserted into the active site loop of thioredoxin showed the same greatly enhanced binding compared to the wild type p53 peptide sequence that we had seen in our earlier analysis of the free peptides, demonstrating the successful transfer of the optimised sequence from phage display to insert protein. Critically, the inhibitory potential of the 15 kD TIP 12/1 protein was the same as that of tetrameric wt p53 and greatly exceeded that achieved by the simple transplantation of the wild type p53 sequence into thioredoxin. When expressed in mammalian cells both aptamers, TIP 12/1 and TIP wt were able to induce p53 dependent transcriptional activation of a reporter gene. The intensity of the effect in microinjection experiments exerted by TIP 12/1 in comparison with TIP wt was proportional to their in vitro inhibitory potential. With these mdm2 binding aptamers we therefore have developed powerful tools to study the biological consequences of disrupting the interaction between mdm2 and p53 in tumour cells. This precise approach of aptamer selection and design describes a road leading from the identification of peptides that are able to disrupt a very specific protein-protein interaction by combinatorial library approaches towards verifying the expected biological effect *in vivo.* This concept in combination with the use of peptide aptamer libraries, for instance expressed and screened the yeast two hybrid system (Colas et al, 1996) should have a great potential for studying networks of protein-protein interactions in eucaryotic cells on the biochemical as well as on the functional level. It certainly now enables us to draw some interesting conclusions about the significance of inhibiting the interaction between mdm2 and p53 in tumour cells.

### Activation of p53 by blocking binding to mdm2

It had been assumed that disrupting the interaction between the two proteins would potentially release transcriptionally active p53 in tumour cells that overexpress mdm2 due to gene amplification. Although our experiments with OSA cells suggest that this is true, these cells doe not seem to be the most susceptible. In contrast, MCF-7 cells and U2-OS cells as well as T22 cells, all of which have hardly detectable mdm2 levels, respond with an immense transcriptional activation of p53 after applying mdm2 inhibitors.

It appears that the interaction of p53 with mdm2 is the prime restraint for p53 activity in all analysed tumour cells. Comparison of the response in different cell lines argues that the release of transcriptionally active p53 is reciprocal to the mdm2 content of the cells. Higher affinity inhibitors or specific targeting of our TIP aptamers to the nucleus could be expected to overcome mdm2 repression of p53 to a much higher degree in cells with amplified mdm2 levels, like OSA cells.

This result also demonstrates that inhibitors of the p53-mdm2 interaction do not interfere with the capacity of p53 to interact with the transcription apparatus. It had been feared that the close similarity between the target site for mdm2 and for the transcription complex on the N-terminus of p53 might mean that any inhibitor of mdm2 binding also blocks the transcriptional apparatus from binding to p53.

### Mdm2 regulates p53 levels

We further show that inhibition of the interaction of p53 with mdm2 leads to a rise in p53 protein levels. This strongly implies that binding of mdm2 to p53 is a requirement for p53 degradation or destabilisation. This conclusion is strongly supported by the finding that the F¹⁹→A mutation blocks mdm2 dependent reductions in p53 levels in transfection studies. Marston et al found that an in frame deletion of conserved box 1 resulted in a mutant p53 that was very stable but retained transcriptional activity (Marston et al, 1994) whilst it is well established that larger N-terminal deletions also stabilise p53. The model that p53 drives the transcription of the key regulator of its own degradation also provides an intensely attractive model for the stability of the p53 protein in cells in which the protein is inactive transcriptionally. This includes cells that contain only mutant p53 such as the majority of human tumour cells and cells infected or transformed by DNA tumour viruses whose products bind an inactivate the transcriptional function of p53. The model argues that these cells lack sufficient functional mdm2 expression to target p53 degradation. The model is completely consistent with our earlier finding that point mutant p53 proteins, inactive in transcription assays, are stable when transfected into p53 null cells but unstable in cells that contain wild type p53 activity (Vojtesek and Lane, 1993; Midgley et al in preparation). If mdm2 targets p53 for degradation, inhibition of this interaction could prevent newly synthesised p53 from being rapidly turned over and so lead to the observed increase in transcriptionally active p53. It would be of great interest to explore if the rise of p53 after UV or other genotoxic damage indeed involves release of the protein from mdm2. This model therefore provides much scope for further study. It contains striking parallels to the model for HPV E6 dependent degradation of p53 and it is a valid speculation that mdm2 may target p53 for ubiquitin dependent degradation. It becomes possible to target other proteins for degradation in a p53 like manner so that they are unstable in normal cells, but stabilised by DNA damage or in cells lacking mdm2 activity by splicing onto them an mdm2 recognition peptide and it is possible to design super active p53 proteins that cannot be targeted for degradation but are transcriptionally active.

Our results suggest that employing inhibitors of the interaction between mdm2 and p53 in tumour cells expressing wt p53 leads the way to induce p53 transcriptional activity. These drugs would potentially mimic the effect of common cancer therapeutic treatments which induce the growth inhibitory and apoptotic properties of p53 by virtue of the induction of DNA damage without requiring the non specific consequences of this DNA damage. Such drugs might show a profound increase in therapeutic effects and avoid the mutagenic and other side effects of conventional therapies.

**Table 1**

| **In vitro inhibitory potential of TIPs compared to free peptides and full length p53** | |
|---|---|
| Inhibitor | IC₅₀in nM |
| TIP wt | 15 000 |
| TIP 12/1 | 300 |
| Trx | no inhibition |
| peptide wt | 2 000 |
| peptide 12/1 | 100 |
| full length p53 | 400 |

### References:

The references cited herein are incorporated by reference in their entirety.

Blaydes, J.P., Gire, V., Rowson, J. M., and Wynford-Thomas, D. (1997). Tolerance of high levels of wild-type p53 in transformed epithelial cells dependent on autho-regulation by mdm-2. Oncogene *14*, 1859-1868 (1997).

Böttger, A., Böttger, V., Garcia Echeverria, C., Chène, P., Hochkeppel, H. K., Sampson, W., Ang, K., Howard, S. F., Picksley, S. M., and Lane, D. P. (1997). Molecular characterization of the hdm2-p53 interaction Molecular characterization of the hdm2-p53 interaction. J-Mol-Biol., in press.

Böttger, V., Böttger, A., Howard, S. F., Picksley, S. M., Chene, P., Garcia Echeverria, C., Hochkeppel, H. K., and Lane, D. P. (1996). Identification of novel mdm2 binding peptides by phage display. Oncogene *13,* 2141-2147.

Chen, C. Y., Oliner, J. D., Zhan, Q., Fornace, A. J., Vogelstein, B., and Kastan, M. B. (1994). Interactions between p53 and MDM2 in a mammalian cell cycle checkpoint pathway. proc Natl Acad Sci U S A 91, 2684-2688.

Colas, P., Cohen, B., Jessen, t., Grishina, I., McCoy, J., and Brent, R. (1996). Genetic selection of peptide aptamers that recognize and inhibit cyclin-dependent kinase 2. Nature *380*, 548-550.

Finlay, C. A. (1993). The mdm-2 oncogene can overcome wild-type p53 suppression of transformed cell growth. Mol-Cell-Biol *13*, 301-306.

Florenes, V. A., Maelandsmo, G. M., Forus, A., Andreassen, A., Myklebost, O., and Fodstad, O. (1994). MDM2 gene amplification and transcript levels in human sarcomas: relationship to TP53 gene status [see comments]. J-Natl-Cancer-Inst *86*, 1297-1302.

Haupt, Y., Barak, y., and Oren, M. (1996). Cell type-specific inhibition of p53-mediated apoptosis by mdm2. *15*, 1596-1606.

Hupp, T. R., Sparks, A., and Lane, D. P. (1995). small peptides activate the latent sequence-specific DNA binding function of p53. Cell *83*, 237-245.

Jones, S. N., Roe, A. E., Donehower, L. A., and Bradley, A. (1995). Rescue of embryonic lethality in Mdm2-deficient mice by absence of p53. Nature *378*, 206-208.

Juven, t., Barak, Y., Zauberman, A., George, D. L., and Oren, M. (1993). wild type p53 can mediate sequence-specific transactivation of an internal promoter within the mdm2 gene. Oncogene *8*, 3411-3416.

Kussie, P. H., Gorina, S., Marechal, V., Elenbaas, B., Moreau, J., Levine, A. J., and Pavletich, N. P. (1996). Structure of the MDM2 oncoprotein bound to the p 53 tumor suppressor transactivation domain [comment]. Science *274*, 948-953.

LaVallie, E. R., DiBlasio, E. A., Kovacic, S., Grant, K. L., Schendel, P. F., McCoy, J. M., and Green, M. r. (1993). A thioredoxin gene fusion expression system that circumvents inclusion body formation in the E. coli cytoplasm. How different eukaryotic transcriptional activators can cooperate promiscuously. Biotechnology (N Y) *11*, 187-193.

Lin, Y. S., and Green, M. R. (1989). similarities between prokaryotic and eukaryotic cyclic AMP-responsive promoter elements. Nature *340*, 656-659.

Lu, X., and Lane, D. P. (1993). differential indication of transcriptionally active p53 following UV or ionizing radiation: defects in chromosome instability syndromes? Cell *75*, 765-778.

Marston, N. J., Crook, T., and Vousden, K. H. (1994). Interaction of p53 with MDM2 is independent of E6 and does not mediate wild type transformation suppressor function. Oncogene *9*, 2707-2716.

Midgeley, C. A., Fisher, C. J., Bartek, J., Vojtesek, B., Lane, D., and Barnes, D. M. (1992). Analysis of p53 expression in human tumours: an antibody raised against human p53 expressed in Escherichia coli. J-Cell-Sci *101*, 183-189.

Momand, J., Zambetti, G. P., Olson, D. C., George, D., and Levine, A. J. (1992). The mdm-2 oncogene product forms a complex with the p53 protein and inhibits p53-mediated transactivation. Cell *69*, 1237-1245.

Montes de Oca Luna, R., Wagner, D. S., and Lozano, G. (1995). Rescue of early embryonic lethality in mdm2-deficient mice by deletion of p53. Nature *378*, 203-206.

Oliner, J. D., Pietenpol, J. A., Thiagalingam, S., Gyuris, J., Kinzler, K. W., and Vogelstein, B. (1993). Oncoprotein MDM2 conceals the activation domain of tumour suppressor p53. Nature *362*, 857-860.

Otto, A., and Deppert, W. (1993). Upregulation of mdm-2 expression in Meth A tumor cells tolerating wild-type p53. Oncogene *8*, 2591-2603.

Picksley, S. M., Vojtesek, B., Sparks, A., and Lane, D. P. (1994). Immunolchemical analysis of the interaction of p53 with MDM2; fine mapping of the MDM2 binding site on p53 using synthetic peptides. Oncogene *9*, 2523-2529.

Renzing, J., and Lane, D. P. (1995). p53-dependent growth arrest following calcium phosphate-mediated transfection of murine fibroblasts. Oncogene *10*, 1865-1868.

Vojtesek, B., and Lane, D. P. (1993). Regulation of p53 protein expression in human breast cancer cell lines. J Cell Sci *105*, 607-612.

Wu, X., Bayle, J. H., Olson, D., and Levine, A. J. (1993). the p53-mdm-2 autoregulatory feedback loop. Genes-Dev 7, 1126-1132.

## Claims

1. Use of an agent having the property of disrupting the binding of p53 and mdm2 or inhibiting the production of mdm2 in a population of cells in the preparation of a medicament for activating p53 for the treatment of conditions associated with low p53 activity,
**characterised in that** said conditions are not associated with overexpression of mdm2.

2. The use of claim 1 wherein the p53 is activated for DNA specific binding and transcription.

3. The use of claim 1 or claim 2 wherein the agent comprises a peptide having an amino acid sequence having at least 70% amino acid sequence identity with a corresponding portion of human p53 which has the property of binding to mdm2.

4. The use of claim 3 wherein the peptide is less than 25 amino acids in length.

5. The use of claim 3 or claim 4 wherein the agent includes the peptide motif FxxxW, where x is any amino acid.

6. The use of claim 1 or claim 2 wherein the agent has the property of binding to one or more regions of mdm2 involved in binding to p53.

7. The use of claim 6 wherein the agent is an antibody which is capable of blocking a p53 binding site of mdm2.

8. The use of claim 1 or claim 2 wherein the agent has the property of competing with mdm2 for binding p53, but does not inhibit a biological activity of p53.

9. The use of claim 8 wherein the agent is an antibody capable of blocking a mdm2 binding site of p53.

10. The use of claim 1 or claim 2 wherein the agent is an antisense oligonucleotide capable of inhibiting the synthesis of mdm2 in the population of cells.

11. The use of any one of the preceding claims wherein the medicament is for the treatment of cancer, a viral condition or other condition associated with non functional p53 or mdm2.

12. A method of activating p53 comprising exposing a population of cells to an agent having the property of disrupting the binding of p53 and mdm2 or inhibiting the production of mdm2 so that p53 in the cells is activated, wherein the cells do not overexpress mdm2, the method being other than a method of treatment of the human or animal body by surgery or therapy or a diagnostic method practised on the human or animal body.

13. The method of claim 12 wherein the p53 is activated for DNA specific binding and transcription.

14. The method of claim 12 or claim 13 wherein the agent is as defined in any one of claims 3 to 10.

15. A method of screening test substances for the property of disrupting the binding of p53 and mdm2 or inhibiting the production of mdm2, the method comprising employing cells which do not overexpress mdm2, the cells being transfected with a reporter construct comprising nucleic acid encoding a reporter polypeptide under the control of promoter elements that respond to the level of p53 activated for DNA specific binding to direct expression of the reporter polypeptide, the method comprising exposing the cells to the candidate substances and detecting the presence of the reporter polypeptide, the method being other than a method of treatment of the human or animal body by surgery or therapy or a diagnostic method practised on the human or animal body.

16. The method of claim 15 wherein tent substances are peptides and the cells are transfected with an expression vector comprising nucleic acid encoding the peptides so that the peptide is expressed in the cells.

17. The method of claim 15 or claim 16 wherein the test substances are expressed as fusion with a peptide capable of displaying the test peptide inserted into the active side loops.

18. The method of claim 17 wherein the peptides are expressed as fusion with thioredoxin.

19. The method of claim 15 wherein the test substances are microinjected into the cells.

20. The method of claim 15 wherein the test substances are coupled to transport molecules so that test substances are transported into the cells.

## Patentansprüche

1. Verwendung eines Mittels, das die Eigenschaft hat, die Bindung von p53 und mdm2 zu zerstören oder die Produktion von mdm2 in einer Zellpopulation zu hemmen, zur Herstellung eines Medikaments zum Aktivieren von p53 zur Behandlung von Affektionen, die mit niedriger p53-Aktivität verbunden sind,
**dadurch gekennzeichnet, dass** die Affektionen nicht mit einer Überexpression von mdm2 verbunden sind.

2. Verwendung nach Anspruch 1, worin das p53 zur DNA-spezifischen Bindung und Transkription aktiviert ist.

3. Verwendung nach Anspruch 1 oder 2, worin das Mittel ein Peptid umfasst, das eine Aminosäuresequenz mit zumindest 70%iger Aminosäuresequenzidentität mit einem entsprechenden Abschnitt von Human-p53 aufweist, der die Eigenschaft hat, sich an mdm2 zu binden.

4. Verwendung nach Anspruch 3, worin das Peptid eine Länge von weniger als 25 Aminosäuren aufweist.

5. Verwendung nach Anspruch 3 oder 4, worin das Mittel das Peptidmotiv FxxxW umfasst, worin x eine beliebige Aminosäure ist.

6. Verwendung nach Anspruch 1 oder 2, worin das Mittel die Eigenschaft hat, eine oder mehrere Regionen von mdm2 zu binden, die an der Bindung an p53 beteiligt sind.

7. Verwendung nach Anspruch 6, worin das Mittel ein Antikörper ist, der zur Blockierung einer p53-Bindungsstelle von mdm2 fähig ist.

8. Verwendung nach Anspruch 1 oder 2, worin das Mittel die Eigenschaft hat, mit mdm2 um die Bindung von p53 zu konkurrieren, aber keine biologische Aktivität von p53 hemmt.

9. Verwendung nach Anspruch 8, worin das Mittel ein Antikörper ist, der zur Blockierung einer mdm2-Bindungsstelle von p53 fähig ist.

10. Verwendung nach Anspruch 1 oder 2, worin das Mittel ein Antisense-Oligonucleotid ist, das zum Hemmen der Synthese von mdm2 in der Zellpopulation fähig ist.

11. Verwendung nach einem der vorangegangenen Ansprüche, worin das Medikament zur Behandlung von Krebs, einer viralen Affektion oder einer anderen Affektion dient, die mit nicht-funktionellem p53 oder mdm2 verbunden ist.

12. Verfahren zur Aktivierung von p53, das das Einwirkenlassen eines Mittels, das die Eigenschaft hat, die Bindung von p53 und mdm2 zu zerstören oder die Produktion von mdm2 zu hemmen, auf eine Zellpopulation, so dass p53 in den Zellen aktiviert wird, wobei die Zellen mdm2 nicht überexprimieren, wobei das Verfahren kein Verfahren zur Behandlung des menschlichen oder eines Tierkörpers durch chirurgischen Eingriff oder Therapie oder am menschlichen oder an einem Tierkörper vorgenommenes Diagnoseverfahren ist.

13. Verfahren nach Anspruch 12, worin das p53 für DNA-spezifische Bindung und Transkription aktiviert wird.

14. Verfahren nach Anspruch 12 oder 13, worin das Mittel wie nach einem der Ansprüche 3 bis 10 definiert ist.

15. Verfahren zum Screenen von Testsubstanzen bezüglich der Eigenschaft, die Bindung von p53 und mdm2 zu zerstören oder die Produktion von mdm2 zu hemmen, wobei das Verfahren den Einsatz von Zellen umfasst, die mdm2 nicht überexprimieren, wobei die Zellen mit einem Reporterkonstrukt transfiziert werden, das Nucleinsäure, die für ein Reporterpolypeptid kodiert, unter der Kontrolle von Promotorelementen umfasst, die auf die Menge an p53 ansprechen, das für DNA-spezifische Bindung aktiviert wurde, um die Expression des Reporterpolypeptids zu steuern, wobei das Verfahren das Einwirkenlassen der Kandidatensubstanzen auf die Zellen und das Nachweisen der Gegenwart des Reporterpolypeptids umfasst, wobei das Verfahren kein Verfahren zur Behandlung des menschlichen oder des Tierkörpers durch chirurgischen Eingriff oder Therapie oder Diagnoseverfahren ist, das am menschlichen oder an einem Tierkörper vorgenommen wird.

16. Verfahren nach Anspruch 15, worin Testsubstanzen Peptide sind und die Zellen mit einem Expressionsvektor transfiziert werden, der Nucleinsäure umfasst, die für die Peptide kodiert, so dass das Peptid in den Zellen exprimiert wird.

17. Verfahren nach Anspruch 15 oder 16, worin die Testsubstanzen als Fusion mit einem Peptid exprimiert werden, das dazu fähig ist, das Testpeptid in die Aktivstellen-Loop insertiert zu präsentieren.

18. Verfahren nach Anspruch 17, worin die Peptide als Fusion mit Thioredoxin exprimiert werden.

19. Verfahren nach Anspruch 15, worin die Testsubstanzen in die Zellen mikroinjiziert werden.

20. Verfahren nach Anspruch 15, worin die Testsubstanzen gekoppelt sind, um Moleküle zu transportieren, so dass Testsubstanzen in die Zellen transportiert werden.

## Revendications

1. Utilisation d'un agent ayant la propriété de rompre la liaison de p53 et mdm2 ou d'inhiber la production de mdm2 dans une population de cellules dans la préparation d'un médicament pour activer p53 pour le traitement de conditions associées à une faible activité de p53,
**caractérisée en ce que** lesdites conditions ne sont pas associées à une surexpression de mdm2.

2. Utilisation de la revendication 1 où p53 est activé pour une liaison et une transcription spécifiques de l'ADN.

3. Utilisation de la revendication 1 ou de la revendication 2 où l'agent comprend un peptide ayant une séquence d'acides aminés ayant au moins 70% d'identité de séquence d'acides aminés avec une portion correspondante de p53 humain qui a la propriété de se lier à mdm2.

4. Utilisation de la revendication 3 où le peptide a moins de 25 acides aminés de long.

5. Utilisation de la revendication 3 ou de la revendication 4 où l'agent comprend le motif peptidique FxxxW, où x est tout acide aminé.

6. Utilisation de la revendication 1 ou de la revendication 2 où l'agent a la propriété de se lier à une ou plusieurs régions de mdm2 impliquées dans la liaison à p53.

7. Utilisation de la revendication 6 où l'agent est un anticorps qui est capable de bloquer un site de liaison de P53 de mdm2.

8. Utilisation de la revendication 1 ou de la revendication 2 où l'agent a la propriété d'entrer en compétition avec mdm2 pour la liaison de p53, mais n'inhibe pas une activité biologique de p53.

9. Utilisation de la revendication 8 où l'agent est un anticorps capable de bloquer un site de liaison de mdm2 de p53.

10. Utilisation de la revendication 1 ou de la revendication 2 où l'agent est un oligonucléotide antisens capable d'inhiber la synthèse de mdm2 dans la population de cellules.

11. Utilisation de l'une quelconque des revendications précédentes où le médicament est pour le traitement d'un cancer, d'une condition virale ou autre condition associée avec p53 ou mdm2 non fonctionnel.

12. Méthode d'activation de p53 consistant à exposer une population de cellules à un agent ayant la propriété de rompre la liaison de p53 et mdm2 ou d'inhiber la production de mdm2 de façon que p53 dans les cellules soit activé, où les cellules ne surexpriment pas mdm2, la méthode étant autre qu'une méthode de traitement du corps humain ou animal par chirurgie ou thérapie ou une méthode de diagnostic mise en pratique sur le corps humain ou animal.

13. Méthode de la revendication 12 où p53 est activé pour une liaison et une transcription spécifiques de l'ADN.

14. Méthode de la revendication 12 ou de la revendication 13, où l'agent est tel que défini dans l'une quelconque des revendications 3 à 10.

15. Méthode de criblage de substances de test pour la propriété de rompre la liaison de p53 et mdm2 ou d'inhiber la production de mdm2, la méthode consistant à employer des cellules qui ne surexpriment pas mdm2, les cellules étant transfectées avec une construction d'un reporteur comprenant un acide nucléique codant pour un polypeptide reporteur sous le contrôle d'éléments promoteurs qui répondent au niveau de p53 activé pour la liaison spécifique de l'ADN pour diriger l'expression du polypeptide reporteur, la méthode consistant à exposer les cellules aux substances candidates et à détecter la présence du polypeptide reporteur, la méthode étant autre qu'une méthode de traitement du corps humain ou animal par chirurgie ou thérapie ou une méthode de diagnostic sur le corps humain ou animal.

16. Méthode de la revendication 15 où les substances de test sont des peptides et les cellules sont transfectées avec un vecteur d'expression comprenant un acide nucléique codant pour les peptides de façon que les peptides soient exprimés dans les cellules.

17. Méthode de la revendication 15 ou de la revendication 16 où les substances de test sont exprimées en tant que fusion avec un peptide capable de présenter le peptide de test inséré dans la boucle du site actif.

18. Méthode de la revendication 17 où les peptides sont exprimés en tant que fusion avec la thiorédoxine.

19. Méthode de la revendication 15 où les substances de test sont microinjectées dans les cellules.

20. Méthode de la revendication 15 où les substances de test sont couplées à des molécules de transport de façon que les substances de test soient transportées dans les cellules.
